# NEUE EUROPÄISCHE PATENTSCHRIFT

(11) **EP 2 448 552 B2**
(45) Veröffentlichungstag und Bekanntmachung des Hinweises auf die Entscheidung über den Einspruch: **04.08.2021**
(45) Hinweis auf die Patenterteilung: 04.03.2015
(21) Anmeldenummer: 09807440.4
(22) Anmeldetag: 02.12.2009
(51) Int. Cl.: A61K 8/64, A61K 8/92, A61Q 19/00, A61K 8/06

(54) **STABILISIERUNG VON HYDROLISIERTEM MILCHPROTEIN DURCH CITRUSÖLE**
STABILIZING HYDROLYSED MILK PROTEIN BY MEANS OF CITRUS OILS
STABILISATION DE PROTÉINE DE LAIT HYDROLYSÉE PAR DES ESSENCES D'AGRUMES

(30) Priorität: 19.12.2008 DE 102008063843
(43) Veröffentlichungstag der Anmeldung: 09.05.2012
(73) Patentinhaber: Beiersdorf AG, 20253 Hamburg (DE)
(72) Erfinder: KRÖPKE, Rainer, 22869 Schenefeldt (DE); KAUFFELDT, Martin, 22303 Hamburg (DE); LÜTTIG, Katja, 20255 Hamburg (DE); KALLMAYER, Volker, 22393 Hamburg (DE)
(86) Internationale Anmeldenummer: PCT/EP2009/008579
(87) Internationale Veröffentlichungsnummer: WO 2010/069463

(56) Entgegenhaltungen:
- EP-A2- 0 296 117
- WO-A2-03/077856
- WO-A2-2004/007520
- DE-A1- 1 492 121
- DE-A1- 19 941 819
- JP-A- 60 258 102
- LI S. et al.: "Chemical Instability of Protein Pharmaceuticals: Mechanism of Oxidation and Strategies for Stabilization?;", Biotechnology and Bioengineering, vol. 48, 1995, pages 490-500,
- MIN D. B. et al.: "Chemistry and Reaction of Singlet Oxygen in Foods", Comprehensive Reviews in Food Science and Food Safety, vol. 1, 2001, pages 58-72,
- H. BROCKMANN et al.: "Bouillon-Geruch der Proteinhydrolysate", Angew. Chem., vol. 11, 1955, page 303,
- REHMAN Z.: "Citrus peel extract - A natural source of antioxidant"", Food Chemistry, vol. 99, 2006, pages 450-454,
- EDRIS A. E.;: ""Pharmaceutical and Therapeutic Potentials of Essential Oils and Their Individual Volatile Constituents: A Review"", Phytotherapy Research, vol. 21, 2007, pages 303-323,
- BAIK J. S. et al.: "Chemical Composition and Biological Acitivities of Essential Oils Extracted form Korean Endemic Citrus Species", J. Microbiol. Biotechnol., vol. 2, February 2008 (2008-02), pages 74-79,
- JEONG S.-K. et al.: ""Effect of Heat Treatment on the Antioxidant Activity of Extracts from Citrus Peels"", J. Agric. Food Chem., vol. 52, 2004, pages 3389-3393,

## Beschreibung

Die Erfindung beschreibt kosmetische oder dermatologische Zubereitung umfassend hydrolysiertes Milchprotein und Citrusöle. Die Citrusöle stabilisieren das hydrolysierte Milchprotein in kosmetischen oder dermatologischen Zubereitungen ohne olfaktorische Veränderungen des Endproduktes in Kauf zu nehmen.

Hydrolsiertes Milchprotein, auch Milk Protein, Hydrolyzed milk protein' bezeichnet, wird in erster Linie aus der Milch von Kühen gewonnen und in Kosmetika, Shampoos, Feuchtigkeitscremes, Conditioners, etc.als auch in vielen Lebensmitteln, Klebstoffen und Farben eingesetzt. Aus der Tiernahrung ist beispielsweise das hydrolysierte Milchprotein Alpha-S1Casein bekannt.

Hydrolysiertes Milchprotein ist auch als Bestandteil von Kosmetika bekannt. Hydrolysiertes Milchprotein wird dabei häufig aufgrund der Feuchtigkeitssspendenden Wirkung und als Filmbildner zugefügt.

Die industrielle Hydrolyse von Mitchproteinen durch technische Peptidasen führt je nach Substratzusammensetzung, eingesetztem Enzympräparat und Hydrolysebedingungen zu einem Peptidgemisch, das hunderte an lang-, mittelund kurzkettigen Peptiden sowie Aminosäuren enthalten kann.

Die DE 102006003924 beschreibt den Einsatz von Proteinhydrolysaten zum Färben und/oder Aufhellen keratinischer Fasern.
Die EP 1635776 offenbart O/W-Emulsionen mit hydrolysierten Milchproteinen und Parfum. Ein besonders hoher Anteil an Citrusölen im Parfum und deren Stabilisierung wird nicht offenbart.
Auch die DE 102006004714 beschreibt Haarbehandlungsmittel mit Proteinhydrolysaten. Eine Kombination mit Citrusölen und insbesondere deren Wirkung als Stabilisator für hydrolysierte Milchproteine wird im Stand der Technik nicht erwähnt. Vielmehr werden, wie auch in der US 20050143267, bereits stabilisierte, d.h. chemisch veränderte Milchproteine, wie - polyquaternium 79 hydrolyzed milk protein - eingesetzt. Dies deutet auf das weiterhin bestehende Verlangen noch Stabilisierungsmöglichkeiten für hydrolysierte Milchproteine hin. Hydrolysiertes Milchprotein enthält größere Anteile Aminosäuren, die auch in ihrer im Protein gebundenen Form oxidationsempfindlich sind. Dazu gehören Serin, Threonin und Tyrosin, die über eine freie OH-Gruppe verfügen. Besonders relevant sind aber Methionin und Cystein, die aufgrund der enthaltenen Schwefelgruppe bei Oxidation zu unerwünschten Gerüchen führen können.

Nachteilig bei hydrolysiertem Milchprotein enthaltenen Kosmetika ist deren Instabilität und die damit verbundenen Geruchsbeeinträchtigungen.

Wünschenswert wäre es, Kosmetika mit Milchproteinen bereit zustellen, die keine Stabilitätsprobleme aufweisen und insbesondere hinsichtlich ihrer olfaktorischen Gestaltung unbeeinflusst bleiben.

Citrusöle sind ätherische Öle, die aus den Schalen von Citrusfrüchten (Bergamotte, Grapefruit, Limette, Mandarine, Orange, Zitrone) gewonnen werden.
Citrusöle bestehen zu einem großen Teil aus Monoterpenkohlenwasserstoffen, hauptsächlich Limonen. Ausnahme bildet Bergamottöl, das nur ca. 40 % enthält. Da die wichtigen geruchs- und geschmacksgebenden Komponenten deshalb nur in verhältnismäßig geringen Konzentrationen vorhanden sind, werden vor allem für den Einsatz als Aromatisierungsmittel sogenannte konzentrierte Citrusöle hergestellt, indem man einen Großteil der unerwünschten unpolaren Terpenkohlenwasserstoffe abtrennt und so den Gehalt der polaren Komponenten erhöht, was technisch durch Destillations-, Verteilungs-, Extraktions- oder Adsorptionsverfahren geschehen kann.
Citrus Parfümöle umfassen insbesondere Orangenöle und Bergamotte-Öle. Citrusöle wie das Bergamotte-Öl enthalten als Hauptbestandteil Terpene. Terpene sind Oligomere des Kohlenwasserstoffs Isopren (2-Methyl-1.3-Butadien). Zu den bekanntesten Terpenen gehören Geraniol, Farnesol, Limonen, Campher und Nerol.
Neben ihrer antimikrobiellen Wirkung ist auch eine antioxidative Wirkung von Terpenen bekannt, beispielsweise von Beta-Carotin, einem Tetraterpen. Chemisch ist die antioxidative Wirkung auf die Sauerstoffbindung an den Doppelbindungen des Isoprens zu erklären.

Die Erfindung umfasst kosmetische oder dermatologische Zubereitungen wie in Anspruch 1 definiert.

Als erfindungsgemäße hydrolysierte Milchproteine sind vorzugsweise chemisch unveränderte Proteine zu verwenden. Insbesondere sind hydrolysierte Milchproteinderivate, wie

Palmitoylderivate oder Quaterne Ammoniumsalzderivate, wie Polyquaternium-79 hydrolyzed milk protein, keine erfindungsgemäßen Proteine. Diese Derivate stellen keine erfindungsgemäß zu stabilisierenden Proteine dar, da diese zumeist bereits als stabil gelten.

Erstaunlicherweise führt der Zusatz an Citrusölen zu einer Stabilisierung der hydrolysierten Milchproteine in den kosmetischen oder dermatologischen Zubereitungen.

Es hat sich herausgestellt, dass die in den Citrusölen enthaltenen Terpene als sogenannte "Opfermoleküle" wirken. Sie verhindern oder vermindern zumindest damit die Oxidation des hydrolysierten Milchproteins und die damit verbundene unerwünschte Geruchsentwicklung im kosmetischen Produkt. Sie tragen damit zu einer verbesserten Lagerstabilität des Produktes enthaltend hydrolysiertes Milchprotein bei.

Die oxidationsempfindliche Aminosäuren in den hydrolysierten Milchproteinen, wie Met 2,7%, Cys 0,4%, Ser 5,9%, Thr 3,8%, Tyr 5,1%, können nun durch den Zusatz an Citrusöl geschützt werden. D.h. die Zubereitung wird stabilisiert.

Um den kosmetischen oder dermatologischen Produkten nicht nur den Citrusduft zu eigen werden zulassen sondern eine olfaktorsiche Gestaltungsmöglichkeit aufrecht zu erhalten, werden die Citrusöle den ansonsten in den Zubereitungen enthaltenen Parfumölen zugesetzt. Die Parfumöle werden somit mit Citrusölen angereichert.

Es werden so einerseits die Milchproteine in der Formulierung stabilisiert und andererseits die Duftwirkung der kosmetischen Zubereitung nicht verfälscht.
Es werden bis zu 30 Gew.% Citrusöl, bezogen auf die Gesamtmasse des Parfumöls, den bestehenden Parfumölen zugefügt. Vorteilhaft umfasst der Anteil an Citrusölen einen Bereich von etwa 8 bis 25 Gew.%, insbesondere 10 bis 20 Gew.%, bezogen auf die auf die Gesamtmasse des Parfumöls.
Der Vorteil ist, dass so der eigentliche Parfumeindruck bestehen bleibt und es nicht nur nach Citrusöl duftet.

Orangenöl und/oder Bergamottöl wird den Parfumölen zugesetzt. Die Hauptbestandteile des Bergamotte-Öls sind Terpene wie Linalylacetat, Linalool, Bergapten, Dihydrocuminalkohol, Nerol, Limonen, Bergaptol, Limettin und Bergamottin.

Die Terpene lassen sich formal als Oligomere des Kohlenwasserstoffs Isopren [2-Methyl-1.3-butadien CH2=C(CH3)-CH=CH2] auffassen und aus C5-Einheiten, Isopentylen- oder Isopreneinheiten [Isoprenoide] zusammensetzen. Je nach Zahl dieser Basiseinheiten teilt man sie in Monoterpene (C10, 2 Isopreneinheiten), Sesquiterpene (C15, 3Isopreneinheiten), Diterpene (C20, 4 Isopreneinheiten), Sesterpene (C25), Triterpene (C30) und Tetraterpene (C40) ein. Die Steroide leiten sich von den Triterpenen ab, Carotinoide besitzen meist Tetraterpen-Struktur.

Hinsichtlich des Anteils an Citrusölen bezogen auf die Gesamtmasse der Zubereitung, liegt dieser im Bereich von 0,01 bis 0,1 Gew.%.

Vorteilhaft sind die erfindungsgemäßen Zubereitungen topisch applizierbare Zubereitungen, insbesondere auf Basis von O/W-Emulsionen. Der Zusatz an Tensiden oder die Anwendung als Wasch- oder Reinigungszubereitung liegt nicht im erfindungsgemäßen Fokus und ist daher vorteilhaft nicht umfasst.

Nachfolgende Beispiele erläutern die erfindungsgemäßen Zubereitungen. Vorteilhaft sind die Zubereitungen als O/W-Emulsion formuliert. Die angegeben Zahlen sind Gewichtsanteile bezogen auf die Gesamtmasse der Zubereitung,

### Beispiele

### O/W-Emulsion

| | 1 | 2 | 3 | 4 | 5 |
|---|---|---|---|---|---|
| Glycerylstearatcitrat | 2,4 | 2,0 | 2,0 | 2,0 | 2,4 |
| Medizinisches Weissöl | --- | 6,0 | 6,0 | 6,0 | --- |
| Myristylmyristat | 3,0 | --- | --- | --- | 3,0 |
| Triclosan | --- | --- | 0,06 | --- | --- |
| Isopropylpalmitat | --- | 3,0 | 3,0 | 3,0 | --- |
| Caprylic/Capric Triglycerid | 4,0 | --- | --- | --- | 4,0 |
| lineares Silikonöl | 2,0 | 1,0 | 1,0 | 1,0 | 2,0 |
| Propylparaben | 0,2 | 0,1 | 0,1 | 0,1 | 0,2 |
| Sorbitanstearat | 0,8 | --- | --- | --- | 0,8 |
| Octyldodecanol | 1,0 | --- | --- | --- | 1,0 |
| Cetearylalkohol | 0,8 | 2,0 | 2,0 | 2,0 | 0,8 |
| Phenoxyethanol | 0,6 | 0,5 | 0,5 | 0,5 | 0,6 |
| Glycerin | 6,5 | --- | --- | --- | 6,5 |
| Polyacrylsäure, Natrium-Salz | 0,19 | 0,19 | 0,19 | 0,19 | 0,19 |
| Xanthan Gum | 0,2 | --- | --- | --- | 0,2 |
| Methylparaben | 0,3 | 0,3 | 0,3 | 0,3 | 0,3 |
| Alkohol | 3,0 | --- | --- | --- | 3,0 |
| Bambusextrakt | 0,1 | 0,1 | 0,1 | 0,1 | 1,0 |
| Hydrolysiertes Milchprotein | 0,05 | 0,05 | 0,05 | 0,05 | 0,5 |
| Glycerylglukosid | 5,0 | 5,0 | 5,0 | 5,0 | 5,0 |
| Parfum | 0,3 | 0,3 | 0,3 | 0,3 | 0,6 |
| Orangenöl "Orange oil sweet expressed" | 0,03 | 0,04 | 0,035 | 0,05 | 0,075 |
| Wasser | ad 100 | ad 100 | ad 100 | ad 100 | ad 100 |

## Patentansprüche

1. Kosmetische oder dermatologische Zubereitung umfassend hydrolisiertes Milchprotein, ein oder mehrere Citrusöle und zusätzlich ein oder mehrere Parfumöle, die die Citrusöle umfassen, **dadurch gekennzeichnet, dass** der Anteil an Citrusölen im Bereich von 0,01 bis 0,1 Gew.%, bezogen auf die auf die Gesamtmasse der Zubereitung, beträgt, die Citrusöle bis zu einem Anteil bis zu 30 Gew.%, bezogen auf die Gesamtmasse an Parfumöl, enthalten sind und als Citrusöle Orangenöl und/oder Bergamotöl gewählt werden.

2. Zubereitung nach einem der vorstehenden Ansprüche in Form einer O/W-Emulsion.

## Claims

1. Cosmetic or dermatological preparation comprising hydrolysed milk protein, one or more citrus oils and additionally one or more perfume oils which comprise the citrus oils, **characterized in that** the fraction of citrus oils is in the range from 0.01% to 0.1% by weight, based on the total mass of the preparation, the citrus oils are present up to a fraction up to 30% by weight, based on the total mass of perfume oil, and orange oil and/or bergamot oil are selected as citrus oils.

2. Preparation according to one of the preceding claims in the form of an O/W emulsion.

## Revendications

1. Préparation cosmétique ou dermatologique comprenant une protéine lactique hydrolysée, une ou plusieurs huiles d'agrumes et en outre une ou plusieurs huiles parfumées qui comprennent les huiles d'agrumes, **caractérisée en ce que** la proportion d'huiles d'agrumes est dans la plage allant de 0,01 à 0,1 % en poids, par rapport à la masse totale de la préparation, les huiles d'agrumes sont contenues en une proportion de jusqu'à 30 % en poids, par rapport à la masse totale d'huile parfumée et l'huile d'orange et/ou l'huile de bergamote sont choisies en tant qu'huiles d'agrumes.

2. Préparation selon l'une quelconque des revendications précédentes, sous la forme d'une émulsion H/E.
